# EUROPEAN PATENT APPLICATION

(11) **EP 3 175 768 A1**
(43) Date of publication of application: **07.06.2017**
(21) Application number: 15848542.5
(22) Date of filing: 28.09.2015
(51) Int. Cl.: A61B 1/00, G02B 23/24

(54) **ENDOSCOPE**

(30) Priority: 06.10.2014 JP 2014205853
(71) Applicant: Olympus Corporation, Tokyo 192-8507 (JP)
(72) Inventor: FUJITANI, Kiwamu, Hachioji-shi, Tokyo 192-8507 (JP)
(74) Representative: Schicker, Silvia
(86) International application number: PCT/JP2015/077284
(87) International publication number: WO 2016/056417

(57) **Abstract**

An endoscope 10 includes a bending portion 16 provided on a distal end side of an insertion portion 11, bending wires 21 and 22 fixed on a distal end side of the bending portion 16, wire insertion portions 23 and 44 into which the bending wires 21 and 22 are inserted in the bending portion 16, and a channel tube 18 inserted and disposed in the insertion portion 11 and including an axial-directional through-hole 18h into which a treatment instrument 60 is inserted. The bending portion 16 includes a bending portion set 20 including a first bending piece set 30 including a plurality of bending pieces 31 and 32 rotatably coupled with each other; a tubular bending tube 40 including a distal end portion rotatably coupled with a proximal end portion of the first bending piece set 30 and capable of bending through a plurality of arrayed slits 41 and 42; and a second bending piece set 50 including a plurality of bending pieces 52 and 53 rotatably coupled with a proximal end portion of the tubular bending tube 40.

## Description

### Technical Field

The present invention relates to an endoscope including a bending portion in an insertion portion.

### Background Art

Recently, an endoscope has been used in, for example, the medical and industrial fields. In the endoscope, an observation optical system for picking up an observation image of an observation site is provided at a distal end portion of an elongated insertion portion.

A bending portion to allow easy insertion into an in-vivo deep part and allow the observation optical system provided at the distal end portion to face in a desired direction is provided on a distal end side of the insertion portion of the endoscope.

The bending portion is configured to perform a bending operation toward a desired direction through a rotational operation on, for example, a rotational knob as a bending operation member provided at an operation portion of the endoscope.

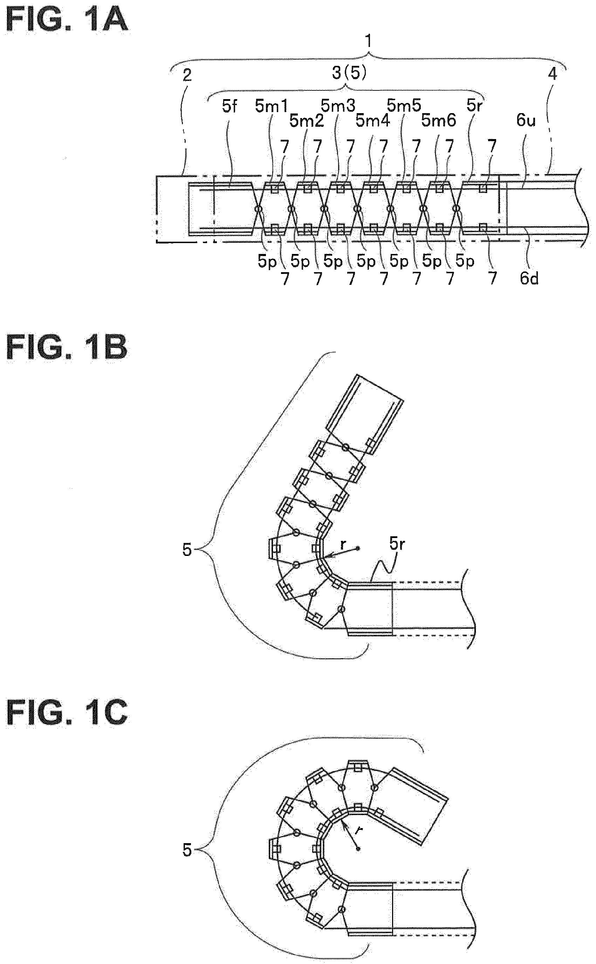

As illustrated in Fig. 1A, an insertion portion 1 is configured to include a distal end rigid portion 2, a bending portion 3, and a flexible tube portion 4 provided juncturally and sequentially from the distal end side. The bending portion 3 is typically configured to include a bending piece set 5, a mesh tube (not illustrated), and a bending rubber (not illustrated).

The bending piece set 5 includes a distal end bending piece 5f of, for example, six intermediate bending pieces 5ml, ... 5m6, and a proximal end bending piece 5r, and the bending pieces are rotatably coupled with each other by a coupling pin 5p and configured to bend upward and downward in two directions.

Note that, the mesh tube covers the bending piece set 5, and the bending rubber is an outer skin as an outermost layer of the bending portion 3 and covers, for example, an outer periphery of the mesh tube.

Reference sign 6u denotes an upper bending wire (hereinafter simply referred to as an upper wire), reference sign 6d denotes a lower bending wire (hereinafter simply referred to as a lower wire), and reference sign 7 denotes a wire guide. The upper wire 6u and the lower wire 6d are respectively inserted in the predetermined wire guides 7.

Note that, the number of intermediate bending pieces is not limited to six, but may be larger or smaller than six. The bending portion 3 is not limited to a configuration that bends upward and downward in two directions, but may be configured to bend upward, downward, leftward, and rightward in four directions, for example.

In the endoscope, when the rotational knob is operated to pull, for example, the upper wire 6u, each of the bending pieces 5f, 5ml, ..., and 5r is rotated about a coupling pin 5p coupling the pieces to each other in a wire pull direction, so that the bending portion 3 bends in the upward direction.

The bending piece set 5 as the bending portion 3 starts bending at the proximal end bending piece 5r illustrated in Fig. 1B. Japanese Patent Application Laid-Open Publication No. 2009-78012 discloses an endoscope in which bending can start on a distal end side and a bending portion being bent can be easily changed back to substantially a straight line, and paragraph [0005] recites that bending starts at a bending piece disposed on a most proximal end side when an angle wire is pulled.

As illustrated in Fig. 1C, when end edges of adjacent bending pieces become contact with each other, the bending piece set 5 becomes in a maximum bending state illustrated with dashed lines, having a bending angle of, for example, 180° or larger. In a state in which the end edges of adjacent bending pieces are in contact with each other, a bending radius is set to r.

Recently, Japanese Patent No. 5444516 discloses use of a bending tube made of a super elastic alloy material in place of a bending piece set.

The bending tube made of a super elastic alloy can achieve a bending portion with a simple configuration by providing, for example, a plurality of partially arc-shaped slits to a tubular member by laser fabrication or the like, without using, for example, a plurality of bending pieces and coupling pins. Setting of a bending radius and adjustment of bendability can be performed by setting intervals and the like of the slits provided to the bending tube as appropriate.

Note that, when the intervals of the slits are set to be small, the bending radius becomes small and the bendability is improved.

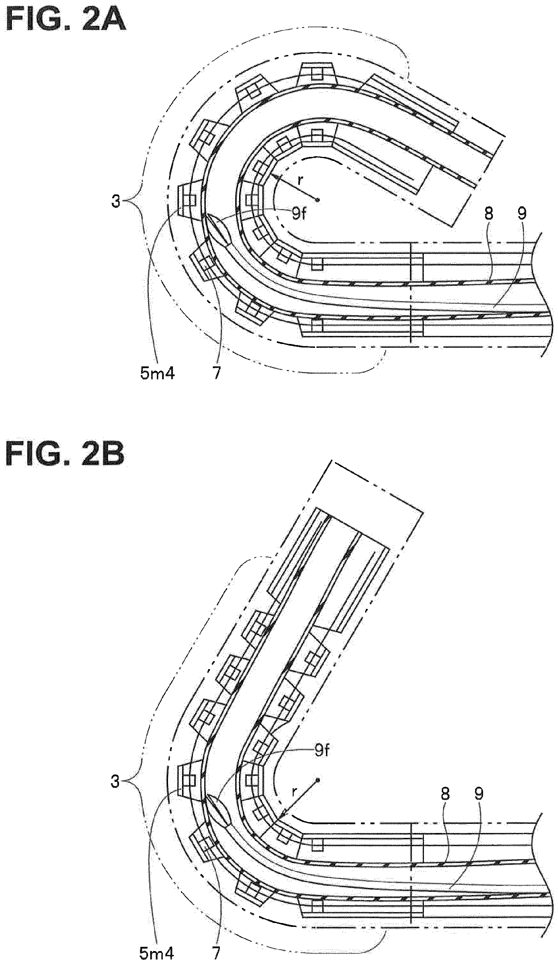

However, before a maximum bending state is reached, in the bending portion 3, the end edges of adjacent bending pieces become in contact with each other at a certain bending angle or larger as illustrated in Fig. 1B, a bending radius of the plurality of bending pieces bent from the proximal end side becomes r as indicated in Fig. 1C. Thus, when a treatment instrument is introduced into a channel tube 8 inserted and disposed in the bending portion 3, a distal end portion 9f of a treatment instrument 9 moving inside of the channel tube 8 contacts a particular wire guide 7 through the channel tube 8 at substantially the same position (near an intermediate bending piece 5m4 in the drawing) inside of the bending portion 3 whether the bending angle of the bending portion 3 is 180° or larger as illustrated in Fig. 2A or 120° as illustrated in Fig. 2B.

As a result, resistance potentially degrades extremely at part of the channel tube 8 inserted and disposed in the bending portion 3.

The present invention is intended to solve the above-described problem and provide an endoscope that prevents degradation of resistance at a particular site of a channel tube inserted and disposed in a bending portion.

### Disclosure of Invention

### Means for Solving the Problem

An endoscope according to an aspect of the present invention includes: a bending portion provided on a distal end side of an insertion portion; a plurality of bending operation wires fixed on a distal end side of the bending portion;
a wire insertion portion into which each of the bending operation wires is inserted in the bending portion; and a tubular body inserted and disposed in the insertion portion and including an axial-directional through-hole into which a treatment instrument is inserted. The bending portion includes a bending portion set including: a first bending piece set that includes a plurality of bending pieces rotatably coupled with each other and a wire insertion portion into which the bending operation wire is inserted to be guided to a proximal end side, a distal end of the bending operation wire being fixed on an inner surface of one of the plurality of bending pieces; a tubular bending tube that is a tubular member including a distal end portion rotatably coupled with a proximal end portion of the first bending piece set, is capable of bending through a plurality of slits arrayed in a longitudinal direction of the tubular member, each slit extending in a circumferential direction, and includes a wire insertion portion into which the bending operation wire is inserted from a distal end side to a proximal end side; and a second bending piece set that includes a plurality of bending pieces including a bending piece rotatably coupled with a proximal end portion of the tubular bending tube and includes a wire insertion portion into which the bending operation wire is inserted from a distal end side to a proximal end side.

The present invention described above can achieve an endoscope that prevents degradation of resistance at a particular site of a channel tube inserted and disposed in a bending portion.

### Brief Description of the Drawings

Fig. 1A is a diagram for description of a bending piece set configured by a plurality of bending pieces juncturally provided;
Fig. 1B is a diagram for description of the bending piece set configured by a plurality of bending pieces juncturally provided;
Fig. 1C is a diagram for description of the bending piece set configured by a plurality of bending pieces juncturally provided;
Fig. 2A is a diagram for description of a defect of contact of a treatment instrument moving inside of a channel tube inserted and disposed in a bending portion being bent with a particular wire guide;
Fig. 2B is a diagram for description of the defect of contact of the treatment instrument moving inside of the channel tube inserted and disposed in the bending portion being bent with a particular wire guide;
Fig. 3 is a diagram for description of an endoscope characterized in the bending portion;
Fig. 4 is a diagram for description of a configuration of a bending portion set included in the bending portion;
Fig. 5 is a longitudinal cross-sectional view for description of the configuration of the bending portion set;
Fig. 6A is a diagram for description of a bending operation of the bending portion set;
Fig. 6B is a diagram for description of the bending operation of the bending portion set;
Fig. 6C is a diagram for description of the bending operation of the bending portion set;
Fig. 6D is a diagram for description of the bending operation of the bending portion set;
Fig. 7A is a diagram for description of a relation between a channel tube inside of the bending portion and a treatment instrument moving inside of the channel tube;
Fig. 7B is a diagram for description of the relation between the channel tube inside of the bending portion and the treatment instrument moving inside of the channel tube;
Fig. 7C is a diagram for description of the relation between the channel tube inside of the bending portion and the treatment instrument moving inside of the channel tube; and
Fig. 7D is a diagram for description of the relation between the channel tube inside of the bending portion and the treatment instrument moving inside of the channel tube.

### Best Mode for Carrying Out the Invention

Embodiments of the present invention will be described below with reference to the accompanying drawings.

Note that, each drawing used in description below is schematically illustrated, and a dimensional relation, scaling, and the like of components are such that different scaling is applied to each configuration element to illustrate each configuration element in a recognizable manner in the drawings, and thus the present invention is not limited only by the number of configuration elements, shapes of the configuration elements, dimensional ratios of the configuration elements, and a relative positional relation between configuration elements illustrated in these drawings.

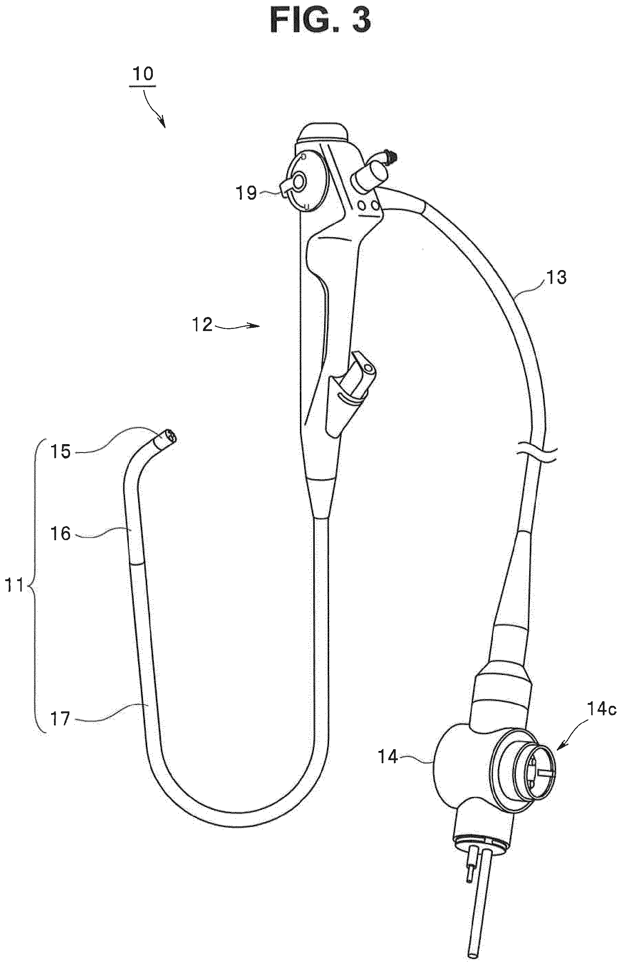

As illustrated in Fig. 3, an endoscope 10 according to the present embodiment includes an insertion portion 11, and an operation portion 12 continuously provided at a proximal end of the insertion portion 11, and a universal cord 13 extends from a side part of the operation portion 12.

An endoscope connector 14 is provided at an end part of the universal cord 13. Reference sign 14c denotes an electric connector portion, and the electric connector portion 14c is detachably connected with an electric cable connected with a video processor (not illustrated) as an endoscope external device. The insertion portion 11 is an elongated tubular member having flexibility, and includes a distal end portion 15, a bending portion 16, and a flexible tube portion 17 provided continuously and sequentially from a distal end side. The distal end portion 15 includes an image pickup apparatus (not illustrated) including an image pickup device, an illumination optical system (not illustrated), and the like.

The bending portion 16 is configured to bend, for example, upward and downward in two directions. The bending portion 16 is configured to perform a bending operation along with a rotational operation on a bending lever 19 as a bending operation member provided to the operation portion 12.

The flexible tube portion 17 is a tubular member configured to passively perform elastic deformation.

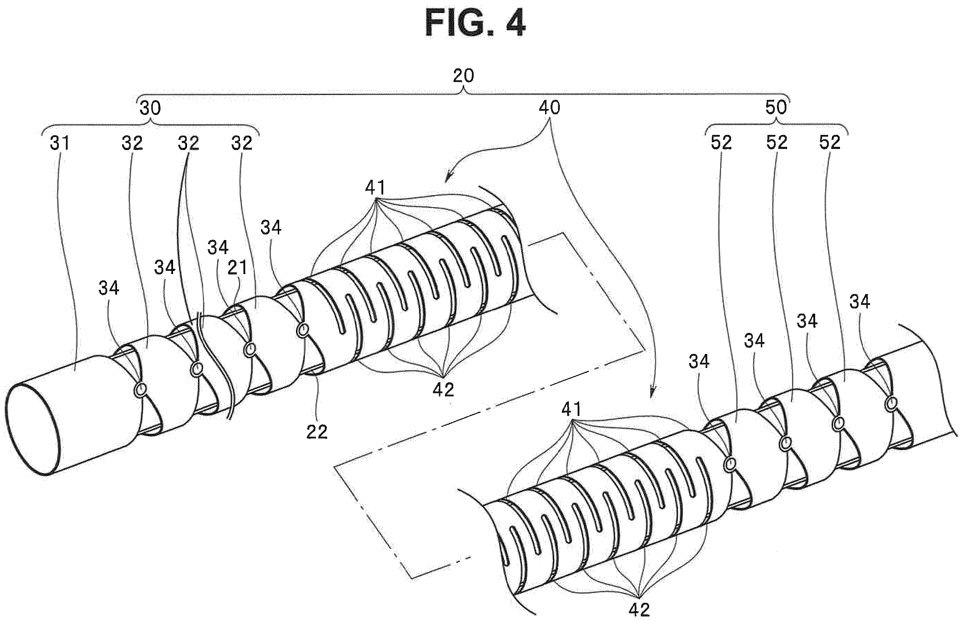

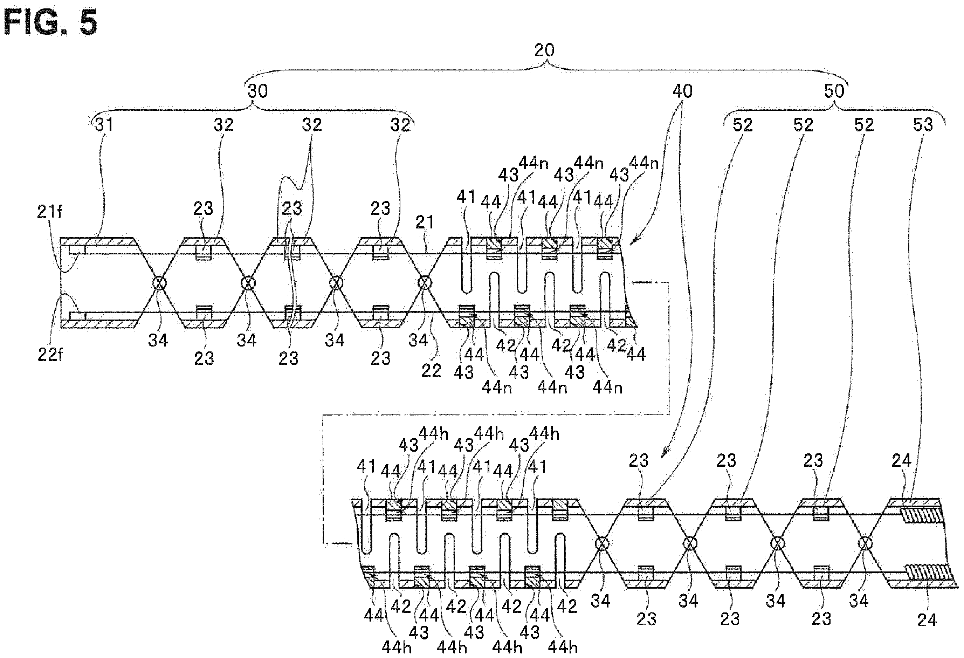

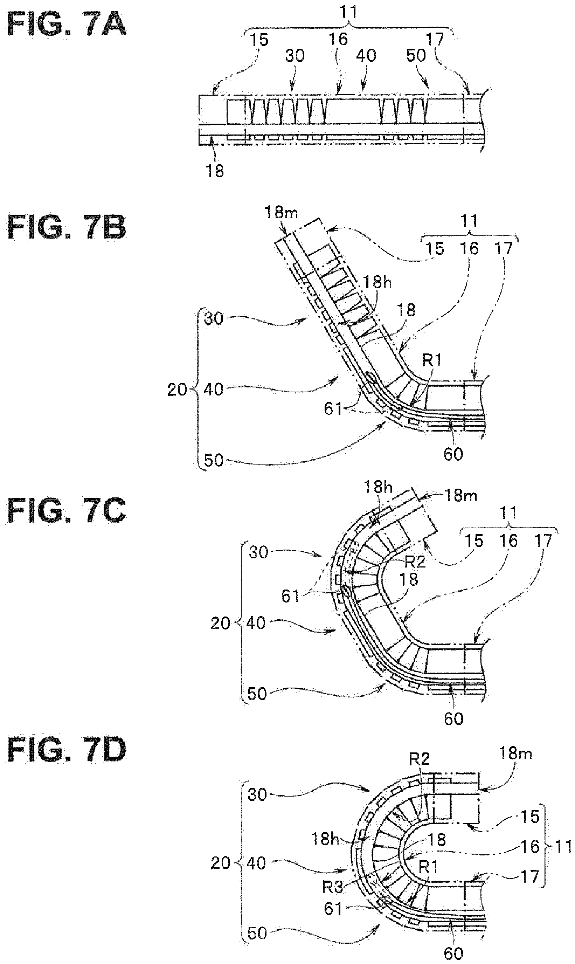

Bending operation wires (reference signs 21 and 22 in Figs. 4 and 5), a channel tube (refer to reference sign 18 in Fig. 7) as a tubular body that forms a treatment instrument insertion channel, various signal lines (not illustrated) extending from the image pickup apparatus, a light guide fiber bundle included in the illumination optical system, an electric cable that supplies electrical power to an LED, or the like is inserted in the insertion portion 11 described above.

Note that, the bending portion 16 is configured to include a bending portion set 20 to be described later, a mesh tube (not illustrated), and a bending rubber (not illustrated).

The mesh tube covers the bending portion set (refer to reference sign 20 in Figs. 4 and 5), and the bending rubber covers the mesh tube. The bending rubber is an outer skin as an outermost layer of the bending portion 16, and is a tubular elastic member made of, for example, elastomer. The bending portion 16 is not limited to a configuration that bends upward and downward in two directions, but may be configured to bend, for example, in four directions of the upward and downward directions and leftward and rightward directions.

The bending portion set 20 included in the bending portion 16 will be described below with reference to Figs. 4 and 5.

As illustrated in Figs. 4 and 5, the bending portion set 20 includes a first bending piece set 30, a tubular bending tube 40, and a second bending piece set 50 provided continuously and sequentially from the distal end side.

The first bending piece set 30 includes a first distal end bending piece 31 and a plurality of first intermediate bending pieces 32, and the bending pieces are rotatably coupled with each other by a coupling pin 34 and are configured to bend upward and downward in two directions.

The distal end side of the first distal end bending piece 31 is integrally fixed to a distal end rigid member (not illustrated) included in the distal end portion 15. A distal end 21 f of the upper bending wire 21 and a distal end 22f of the lower bending wire 22 are fixed at predetermined positions on upper and lower parts, respectively, of an inner peripheral surface of the first distal end bending piece 31.

Wire receivers 23 as wire insertion portions into which the upper bending wire 21 and the lower bending wire 22 are respectively inserted are provided at predetermined positions on upper and lower parts of an inner peripheral surface of each of the plurality of first intermediate bending pieces 32. The upper bending wire 21 and the lower bending wire 22 are inserted into the wire receivers 23 and guided to a proximal end side.

The tubular bending tube 40 is a pipe member made of a super elastic alloy. The tubular bending tube 40 is rotatably coupled with the first intermediate bending pieces 32 on a most proximal end side of the first bending piece set 30 through the coupling pin 34.

Note that, examples of the super elastic alloy member include Ni-Ti, β titanium alloy, and 64 titanium alloy.

A plurality of partially arc-shaped slits (hereinafter simply referred to as slits) 41 and 42 are arrayed in the tubular bending tube 40. The tubular bending tube 40 includes the plurality of arrayed slits 41 and 42 and is configured to bend upward and downward in two directions.

The slits 41 and 42 are each formed, for example, at a spread angle of 200° with respect to a central axis in a circumferential direction on an outer peripheral surface of the tubular bending tube 40. The first slit 41 and the second slit 42 adjacent to each other are separated from each other at a predetermined distance in an axial direction and at positions shifted from each other by 180° in the circumferential direction.

These plurality of slits 41 and 42 are formed, for example, by laser fabrication.

A plurality of guide fixation holes 43 are formed at predetermined positions of upper and lower parts of the tubular bending tube 40. A wire guide 44 is fixed to each guide fixation hole 43.

The wire guide 44 is a wire insertion portion provided with wire insertion holes 44h into which the upper bending wire 21 and the lower bending wire 22 are respectively inserted from the distal end side toward the proximal end side. The second bending piece set 50 includes a plurality of second intermediate bending pieces 52, and a second proximal end bending piece 53, and the second intermediate bending piece 52 disposed on the distal end side among the plurality of second intermediate bending pieces 52 functions as a second distal end bending piece, and is rotatably coupled with a proximal end portion of the tubular bending tube 40 through the coupling pin 34. The bending pieces are rotatably coupled with each other through the coupling pin 34 and configured to bend upward and downward in two directions.

The proximal end side of the second proximal end bending piece 53 is integrally fixed to a coupling tube (not illustrated) provided on the distal end side of the flexible tube portion 17. Wire guides 23 as wire insertion portions into which the upper bending wire 21 and the lower bending wire 22 are respectively inserted are provided at predetermined positions on upper and lower parts of an inner peripheral surface of the second intermediate bending piece 52.

Note that, reference sign 24 denotes each of coil pipes into which the upper bending wire 21 and the lower bending wire 22 are respectively inserted. Instead of the coil pipes 24, the wire guides 23 may be provided to the second proximal end bending piece 53.

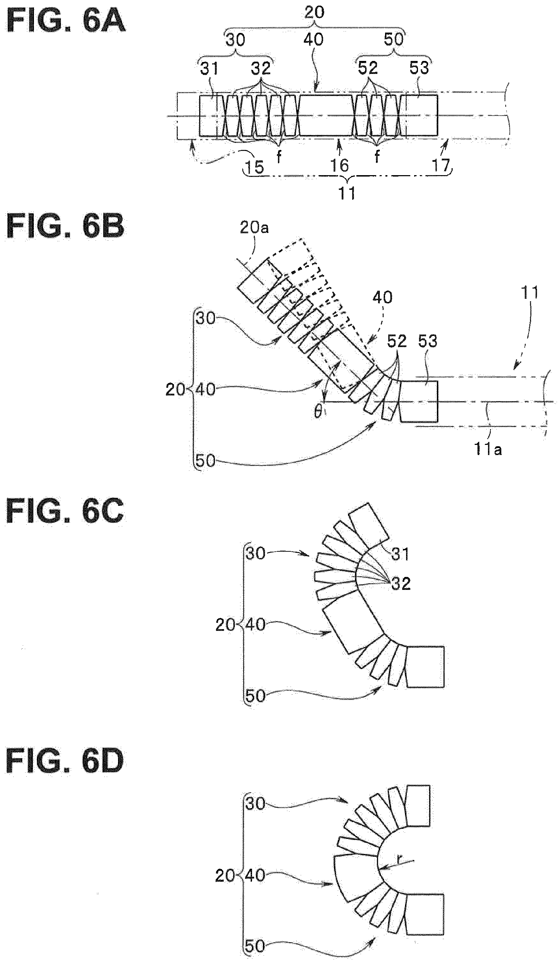

As illustrated in Fig. 6D, axial-directional lengths and end-edge tilt angles of the bending pieces 31 and 32 and the bending pieces 52 and 53 are set such that the bending portion set 20 has a bending radius of r in a maximum bending state. As illustrated in Fig. 6A, the bending piece sets 30 and 50 are each configured to include predetermined numbers of the intermediate bending pieces 32 and 52. In addition, the slits 41 and 42 are provided such that the tubular bending tube 40 has a bending radius of R in a maximum bending state. Note that, illustrations of the slits 41 and 42 are omitted in Fig. 6. In Fig. 6A, reference sign f denotes end edges.

As illustrated in Fig. 6B, as for the second bending piece set 50, the end-edge tilt angle of each bending piece and the number of the second intermediate bending pieces 52 are sets such that an intersecting angle θ between a longitudinal axis 11a of the insertion portion 11 and a bending portion set longitudinal axis 20a is smaller than, for example, 60° when the end edges of adjacent bending pieces are in contact with each other.

Intervals, widths, and circumferential-directional spread angles of the slits 41 and 42 are set such that the tubular bending tube 40 is harder to bend than the second bending piece set 50, in other words, such that a bending stiffness of the tubular bending tube 40 is larger than a bending stiffness of the second bending piece set 50.

Note that, a bending stiffness of the first bending piece set 30 is equivalent to the bending stiffness of the second bending piece set 50.

An effect of the bending portion 16 provided in the insertion portion 11 will be described.

As illustrated in Fig. 6A, the bending portion 16 provided in the insertion portion 11 is normally in a straight state, and the bending portion set 20 is in a straight-line state.

The bending lever 19 is operated by an operator to pull, for example, the upper bending wire 21. Then, along with the pulling of the upper bending wire 21, bending starts at the second proximal end bending piece 53 of the second bending piece set 50 since the tubular bending tube 40 is configured to be harder to bend than the second bending piece set 50.

Specifically, the second intermediate bending piece 52 coupled with the second proximal end bending piece 53 through the coupling pin 34 (no reference sign provided in Fig. 6), and the second intermediate bending piece 52 coupled with the second intermediate bending piece 52 through the coupling pin 34 are rotated in the wire pull direction, and the bending portion 3 is gradually bent upward.

Then, as illustrated in Fig. 6B, the end edges of the adjacent bending pieces 53 and 52, and the end edges of the bending pieces 52 and 52 come in contact with each other, thereby achieving a most bent state of the second bending piece set 50.

When the bending lever 19 is further operated by the operator, the upper bending wire 21 is further pulled, and the tubular bending tube 40 coupled with the second bending piece set 50 through the coupling pin 34 is rotated in the wire pull direction as illustrated with dashed lines in Fig. 6B, so that the end edge of the tubular bending tube 40 on the proximal end side becomes in contact with the end edge of the second intermediate bending piece 52, and a bending angle of the bending portion becomes large.

When the bending lever 19 is further operated by the operator while the end edge of the tubular bending tube 40 on the proximal end side is in contact with the end edge of the second intermediate bending piece 52, the bending portion set 20 is bent as illustrated in Figs. 6C and (D.

As illustrated in Fig. 6C, along with the pulling of the upper bending wire 21, the first bending piece set 30 having a bending stiffness equivalent to the bending stiffness of the second bending piece set 50 starts bending at the first intermediate bending pieces 32. Then, the end edges of the adjacent bending pieces 32 and 32, and the end edges of the bending pieces 32 and 31 are in contact with each other, thereby achieving a most bent state of the first bending piece set 30.

Thereafter, along with the pulling of the upper bending wire 21, the tubular bending tube 40 starts bending on the proximal end side. Then, as illustrated in Fig. 6D, the bending portion set 20 becomes the maximum bending state having the bending radius r.

A treatment instrument that moves inside of an axial-directional through-hole 18h of the channel tube 18 inserted and disposed in the bending portion set 20 being bent as described above will be described with reference to Fig. 7A-(D.

As illustrated in Fig. 7A, the channel tube 18 is inserted and disposed in the first bending piece set 30, the tubular bending tube 40, the second bending piece set 50, and the flexible tube portion 17. Along with a change in a bending shape of the bending portion 16 described above, an insertion-disposition state of the channel tube 18 is deformed from a straight-line shape to a curved-line shape.

As illustrated in Fig. 7B, in a state in which the second bending piece set 50 is bent, the channel tube 18 is deformed mainly in the second bending piece set 50. In the most bent state of the second bending piece set 50, a first curved portion R1 is formed in the channel tube 18 near the second bending piece set 50.

In the first curved portion R1 formed in the second bending piece set 50 in which predetermined bending pieces are arrayed, a distal end portion 61 of a treatment instrument 60 that is moved inside of the channel tube 18 is smoothly moved toward a channel opening 18m without contacting the wire guides 23 through the channel tube.

In a state in which the end edge of the tubular bending tube 40 on the proximal end side is made contact with the end edge of the second intermediate bending piece 52 so that the bending angle becomes large, a shape of the curved portion inside of the second bending piece set 50 does not change largely. Thus, the treatment instrument is moved toward the channel opening 18m, smoothly passing through inside of the curved portion of the second bending piece set 50.

When the first bending piece set 30 is bent in addition to the second bending piece set 50, in the channel tube 18, a second curved portion R2 is formed in the first bending piece set 30 as illustrated in Fig. 7C in addition to the first curved portion R1 in the second bending piece set 50.

Note that, the second curved portion R2 is formed before the first bending piece set 30 comes to the most bent state, in other words, when the end edges of the plurality of first intermediate bending pieces 32 on the proximal end side are in contact with each other. In a state in which the second curved portion R2 is formed in the first bending piece set 30, the shape of the curved portion inside of the second bending piece set 50 does not largely change.

Thus, as illustrated in Fig. 7C, the treatment instrument 60 moving inside of the channel tube 18 smoothly passes through the first curved portion R1. Then, the distal end portion 61 of the treatment instrument 60 is moved toward the channel opening 18m while being in contact with the wire guides 23 through the channel tube near the second curved portion R2.

When the tubular bending tube 40 is bent in addition to the second bending piece set 50 and the first bending piece set 30, in the channel tube 18, a third curved portion R3 is formed in the tubular bending tube 40 as illustrated in Fig. 7D in addition to the first curved portion R1 inside of the second bending piece set 50 and the second curved portion R2 inside of the first bending piece set 30.

Note that, the third curved portion R3 is formed before the tubular bending tube 40 comes to the most bent state, in other words, when opening edges of the plurality of first slits 41 on the proximal end side are in contact with each other.

Then, when the third curved portion R3 is formed in the channel tube 18 inside of the tubular bending tube 40, the treatment instrument 60 moving inside of the channel tube 18 is moved toward the channel opening 18m as illustrated in Fig. 7D while being in contact with the wire guides 23 through the channel tube near a boundary between the first curved portion R1 and the third curved portion R3.

That is, in a state in which the first curved portion R1 is formed, the treatment instrument 60 smoothly moves inside of the channel tube 18. However, in a state in which the second curved portion R2 is formed in addition to the first curved portion R1, the treatment instrument 60 moves toward the channel opening 18m while being in contact with the wire guides 23 through the channel tube near the second curved portion R2. In a state in which the third curved portion R3 is formed in addition to the first curved portion R1 and the second curved portion R2, the treatment instrument 60 moves toward the channel opening 18m while being in contact with the wire guides 23 through the channel tube near the boundary between the first curved portion R1 and the third curved portion R3.

As described above, the bending stiffness of the tubular bending tube 40 is set to be larger than the bending stiffness of the first bending piece set 30 and the bending stiffness of the second bending piece set 50, and then the first bending piece set 30, the tubular bending tube 40, and the second bending piece set 50 of the bending portion set 20 included in the bending portion 16 are provided continuously and sequentially from the distal end side. As a result, the second bending piece set 50, the first bending piece set 30, and the tubular bending tube 40 in the bending portion set 20 are sequentially bent without regularly bending from the proximal end side along with pulling of a bending wire, and thus the distal end portion 61 of the treatment instrument 60 moving inside of the channel tube 18 can be prevented from contacting the wire guides 23 through the channel tube at a particular place inside of the bending portion 16. As a result, a defect of degradation of a resistance at a particular site of the channel tube 18 is removed.

The present invention is not limited only to the above-described embodiment, but various modifications may be performed without departing from the scope of the invention.

The present application is made by using Japanese Patent Application No. 2014-205853 filed on October 6, 2014 as a basis for priority claim, and the above-described content is incorporated in the present specification, claims, and drawings by reference.

## Claims

1. An endoscope comprising:
a bending portion provided on a distal end side of an insertion portion;
a plurality of bending operation wires fixed on a distal end side of the bending portion;
a wire insertion portion into which each of the bending operation wires is inserted in the bending portion; and
a tubular body inserted and disposed in the insertion portion and including an axial-directional through-hole into which a treatment instrument is inserted,
wherein the bending portion includes a bending portion set including: a first bending piece set that includes a plurality of bending pieces rotatably coupled with each other and a wire insertion portion into which the bending operation wire is inserted to be guided to a proximal end side, a distal end of the bending operation wire being fixed on an inner surface of one of the plurality of bending pieces; a tubular bending tube that is a tubular member including a distal end portion rotatably coupled with a proximal end portion of the first bending piece set, is capable of bending through a plurality of slits arrayed in a longitudinal direction of the tubular member, each slit extending in a circumferential direction, and includes a wire insertion portion into which the bending operation wire is inserted from a distal end side to a proximal end side; and a second bending piece set that includes a plurality of bending pieces including a bending piece rotatably coupled with a proximal end portion of the tubular bending tube and includes a wire insertion portion into which the bending operation wire is inserted from a distal end side to a proximal end side.

2. The endoscope according to claim 1, wherein a bending stiffness of the tubular bending tube is set to be larger than a bending stiffness of the second bending piece set.

3. The endoscope according to claim 1, wherein the tubular bending tube is a super elastic pipe member.

4. The endoscope according to claim 3, wherein the super elastic pipe member is made of Ni-Ti.
